# EUROPEAN PATENT SPECIFICATION

(11) **EP 1 194 107 B1**
(45) Date of publication and mention of the grant of the patent: **30.10.2002**
(21) Application number: 00941940.9
(22) Date of filing: 30.06.2000
(51) Int. Cl.: A61H 39/00

(54) **A HANDHELD PIEZOELECTRIC ACUPUNCTURE STIMULATOR**
PIEZOELEKTRISCHES REIZSTROMHANDGERÄT ZUR AKUPUNKTUR
STIMULATEUR PIEZO-ELECTRIQUE PORTATIF DESTINE A L'ACUPUNCTURE

(30) Priority: 02.07.1999 DK 99251
(43) Date of publication of application: 10.04.2002
(73) Proprietor: NODSKOV, Preben, DK-2960 Rungsted Kyst (DK)
(72) Inventor: NODSKOV, Preben, DK-2960 Rungsted Kyst (DK)
(74) Representative: Raffnsoee, Knud Rosenstand
(86) International application number: DK0000355
(87) International publication number: WO01001920

(56) References cited:
- DE-A1- 3 121 254
- DE-A1- 4 026 820
- GB-A- 1 448 644

## Description

The present invention relates to a handheld piezoelectric acupuncture stimulator with a pen-like, substantially electrically insulating exterior casing, at one end of which an actuator button is mounted, while the other end is provided with a contact pin retracted from an end surface intended for contact with the skin in an acupuncture zone, said contact pin being connected with a first electrode of a piezoelectric converter, the second electrode of which is in electrical connection, on one hand, with a hand contact and is mechanically operable, on the other hand, by means of a spring-loaded impact hammer operated by the actuator button for generation of a high-voltage electric pain relieving pulse with a low energy content.

From DE-A1-40 26 820 an acupuncture stimulator of this kind is known, in which the piezoelectric converter and a comparatively long contact pin connected with its first electrode are arranged in their respective electrically insulating casings, surrounded by an electrically conductive metallic exterior casing and a likewise metallic treatment head with an end surface designed for contact with the skin, respectively. The impact hammer with accompanying actuator compression spring is accommodated in a bore in the comparatively elongate actuator button, while the return spring is mounted between recessed shoulder surfaces on the actuator button and an intermediate piece arranged around the piezoelectric converter between the actuator button and the insulating casing.

The considerable number of fairly small individual components in this known stimulator complicates its manufacture and mounting, and the design with an electrically conducting exterior casing and treatment head entails a less satisfactory insulation of the high-voltage electrode of the piezoelectric converter and may impair the efficiency of the stimulator.

These drawbacks are remedied by the invention by a design of a stimulator of the kind defined, which is characterized in that the piezoelectric converter is mounted together with said first and second electrodes and said impact hammer with associated spring system, which comprises an actuator compression spring and a return spring, in a common electrically insulating interior casing designed for form-fit mounting in the exterior casing with said contact pin being retained with a comparatively short, protruding length at one end of the interior casing, at the other end of which a longitudinally displaceable impact hammer actuator is mounted, said actuator being mechanically connected with the actuator button, whereby the electric connection between the second electrode of the piezoelectric converter and said hand contact comprises a leaf spring contact, which projects through the interior casing and extends between said interior casing and the exterior casing for establishing contact with the hand contact, which is designed as a contact ring.

The leaf spring contact is preferably provided with a bent end portion fixed in a recess at the free edge of an end member of the exterior casing, said end member serving as a support for the contact ring. In this way it becomes possible in a simple manner by dimensioning the bent end portion of the leaf spring contact to obtain an accurate fixing of the spark distance between the end of the contact pin and the end portion of the stimulator intended for skin contact.

Owing to the fact that the handheld stimulator is designed for operation of the actuator button by the thumb, an accurate positioning of the exterior contact ring for establishing contact with the user's forefinger may be obtained by mounting the actuator button in a top member placed in extension of the exterior casing and the contact ring and having a protruding abutment as support for the user's forefinger knuckle, when the actuator button is operated by the thumb.

The invention will now be explained in detail in the following with reference to the accompanying drawings, in which

Figs 1 and 2 show an embodiment of a piezoelectric acupuncture stimulator according to the invention in mounted condition, and an exploded view of its main components, respectively, and

Fig. 3 shows more schematically the component parts mounted in the interior casing of the stimulator.

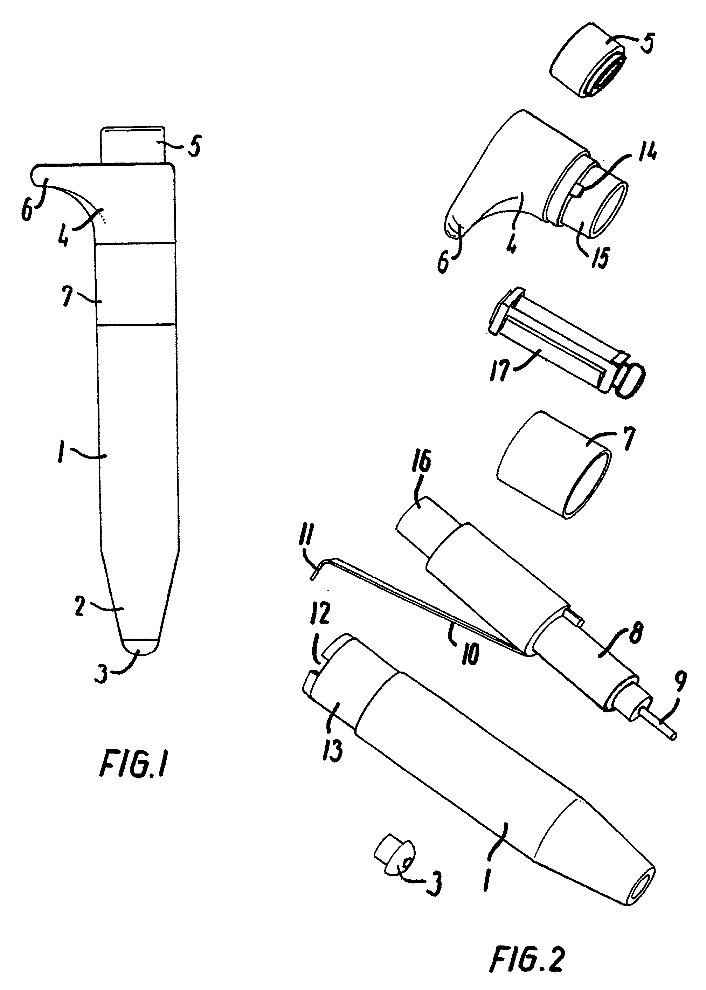

Seen from outside, the handheld piezoelectric acupuncture stimulator comprises, as shown in Fig. 1, an electrically insulating exterior casing 1 of a suitable plastics material, for instance nylon, with a substantially conical end portion 2 in connection with a treatment head 3 designed for contact with the skin in an acupuncture point.

At the opposite end, in extension of the exterior casing 1, a likewise electrically insulating top member 4 is provided, in which an actuator button 5 is mounted. Since the stimulator is designed for operation of the actuator button by the thumb, the top member 4 is designed with a protruding abutment 6, which during use is placed in abutment against the forefinger knuckle and thereby positions a contact ring 7 placed between the top member 4 and the exterior casing 1 in engagement with the user's forefinger.

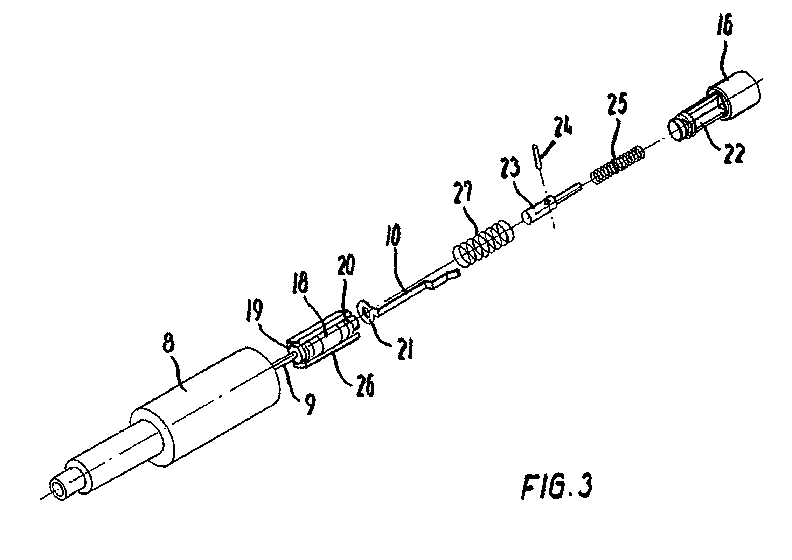

The active components of the stimulator, which will be described below with reference to Fig. 3, are according to the invention, as shown in Fig. 2, mounted in an electrically insulating interior casing 8, from one end of which the contact pin 9 protrudes with a comparatively short length, said contact pin being adapted to transfer the pain relieving pulses produced by the stimulator by spark formation.

The interior casing 8 is designed for form-fit mounting in the exterior casing 1, the end of the contact pin 9 being somewhat retracted from the skin abutment formed by the treatment head 3 for determining a well-defined spark distance.

As likewise shown in Fig. 2, the electric connection between the earth electrode of the stimulator and the contact ring 7 is established by means of a leaf spring contact 10, which is passed through the interior casing 8 and extends between the interior casing and the exterior casing 1. The spring leaf contact 10 ends in a bent portion 11, which, for fixation of the position of the interior casing 8 in the exterior casing 1, is brought into engagement with a recess 12 at the edge of an end portion 13 of the exterior casing 1 designed to support the contact ring 7. The bent end portion 11 of the leaf spring contact 10 is retained in the recess 12 by means of a protruding cam 14 on an end member 15 of the top member 4 adapted to be inserted into the end member 13.

At the opposite end of the interior casing 8 relative to the contact pin 9, a longitudinally displaceable actuator 16 is provided for the mechanical actuation of the piezoelectric converter. In the mounted condition, the actuator 16 is actuated by the actuator button 5 via a profiled pressure member 17 mounted in the top member 4.

The active components of the stimulator, which are mounted in the interior casing 8, comprise, as shown in Fig. 3, both the piezoelectric converter 18 with a first electrode 19 in connection with the contact pin 9 protruding from one end of the interior casing 8 and a second electrode 20 in electrical connection with a contact ring 21 provided at one end of the leaf spring contact 10, and the actuating mechanism designed for mechanical actuation of the converter 18, said mechanism comprising the longitudinally displaceable actuator 16 protruding from the opposite end of the interior casing 8 and having a guide 22 for an impact hammer 23 with a transverse blocking pin 24 and an actuator compression spring 25 and a return spring 27 positioned between the guide 22 and a holder 26 for the converter 18.

The mechanical function of the components shown in Fig. 3 is known per se and has the effect that by a longitudinal displacement of the actuator 16 in the interior casing 8 caused by operation of the actuator button 5, compression of the actuator compression spring 25 occurs at first, which spring by the release of the impact hammer 26 induced by the movement of the actuator with big force shoots the impact hammer towards the second electrode 20 of the converter 18. By the resulting instantaneous compression of the converter 18, the electrical pain relieving pulse with high voltage and comparatively low energy content is generated, for instance at 15,000 volt and 6 µA.

The pain relieving effect of the stimulator resides, as known per se, in that by the spark transfer of the pulse thus generated to an acupuncture point at the place, which is to be relieved from pain, an actuation of the body's own endorphin pain relieving system is supposed to take place.

## Claims

1. A handheld piezoelectric acupuncture stimulator with a pen-like, exterior casing (1), at one end of which an actuator button (5) is mounted, while the other end is provided with a contact pin (9) retracted from an end surface (3) intended for contact with the skin in an acupuncture zone, said contact pin being connected with a first electrode (19) of a piezoelectric converter (18), the second electrode (20) of which is in electrical connection, on one hand, with a hand contact (7) and is mechanically operable, on the other hand, by means of a spring-loaded impact hammer (23) operated by the actuator button (5) for generation of a high-voltage electric pain relieving pulse with a low energy content, **characterized in that** the piezoelectric converter (18) is mounted together with said first and second electrodes (19, 20) and said impact hammer (23) with associated spring system, which comprises an actuator compression spring (25) and a return spring (27), in a common electrically insulating interior casing (8) designed for form-fit mounting in the substantially electrically insulating exterior casing (1) with said contact pin (9) protruding at one end of the interior casing (8), at the other end of which a longitudinally displaceable impact hammer actuator (16) is mounted, said actuator being mechanically connected with the actuator button (5), whereby the electric connection between the second electrode (20) of the piezoelectric converter (18) and said hand contact (7) comprises a leaf spring contact (10), which projects through the interior casing (8) and extends between said interior casing and the exterior casing (1) for establishing contact with the hand contact (7), which is designed as a contact ring.

2. An acupuncture stimulator according to claim 1, **characterized in that** the leaf spring contact (10) with a bent end portion (11) is fixed in a recess (12) at the free edge of an end member (13) of the exterior casing (1), said end member serving as a support for the contact ring (7).

3. An acupuncture stimulator according to claim 1 or 2, **characterized in that** the actuator button (5) is mounted in a top member (4) placed in extension of the exterior casing (1) and the contact ring (7) and having a protruding abutment (6) as support for the forefinger knuckle of the user, when the actuator button (5) is operated by the thumb.

## Patentansprüche

1. Handbetätigter piezoelektrischer Akupunktur-Stimulator mit einem stabähnlichen hülsenförmigen Aussengehäuse (1), an dessen einem Ende ein Aktivierungsknopf (5) montiert ist, während an dem anderen Ende ein von einer Endfläche (3) zurückversetzter Kontaktstift (9) zur Berührung mit der Haut in einer Akupunkturzone vorgesehen ist, welcher Kontaktstift mit einer ersten Elektrode (19) eines piezoelektrischen Wandlers (18) verbunden ist, dessen zweite Elektrode (20) teils mit einem Handkontakt (7) in elektrischer Verbindung steht, und teils mittels eines durch den Aktivierungsknopf (5) betätigten federbelasteten Schlaghammer (23) zur Erzeugung eines schmerzlindernden, elektrischen Hochspannungsimpulses mit niedrigem Energieinhalt mechanisch bedienbar ist, **dadurch gekennzeichnet, dass** der piezoelektrische Wandler (18) zusammen mit erwähnter erster und zweiter Elektrode (19, 20) und erwähntem Schlaghammer (23) mit zugehörigem Federsystem, das eine Aktivierungsdruckfeder (23) und eine Retourfeder (27) umfasst, in einem gemeinsamen elektrisch isolierendem Innengehäuse (8) montiert ist, welches Innengehäuse für formangepasstes Montieren in dem im wesentlichen elektrisch isolierendem Aussengehäuse (1) vorgesehen ist, wobei erwähnter Kontaktstift (9) an dem einen Ende des Innengehäuses (8) herausragt, und an dem anderen Ende des Gehäuses eine in Längsrichtung verschiebbare Schlaghammerbetätigung (16) montiert ist, welche Betätigung mit dem Aktivierungsknopf (5) mechanisch verbunden ist, und die elektrische Verbindung zwischen der zweiten Elektrode (20) des piezoelektrischen Wandlers (18) und dem Handkontakt (7) einen Blattfederkontakt (10) umfasst, der durch das Innengehäuse (8) herausgeführt wird und zwischen dem Innengehäuse und dem Aussengehäuse (1) verläuft, zwecks Kontaktherstellung mit dem als Kontaktring ausgebildeten Handkontakt (7).

2. Akupunktur-Stimulator nach Anspruch 1, **dadurch gekennzeichnet, dass** der Blattfederkontakt (10) mit einem ungebogenen Endteil (11) in einer Aussparung (12) in der freien Kante eines als Stütze für den Kontaktring (7) dienenden Endteils des Aussengehäuses (1) befestigt ist.

3. Akupunktur-Stimulator nach Anspruch 1 oder 2, **dadurch gekennzeichnet, dass** der Aktivierungsknopf (5) in einem in Verlängerung des Aussengehäuses (1) und des Kontaktringes (7) plazierten Oberteil (4) montiert ist, der eine vorstehende Anlage als Stütze für den Zeigefingerknöchel des Benutzers bei Bedienung des Aktivierungsknopfes (5) durch den Daumen bildet.

## Revendications

1. Stimulateur piézoélectrique portatif destiné à l'acupuncture avec un fourreau extérieur semblable à une plume (1), à l'un bout de laquelle est monté un bouton-poussoir d'actionnement (5), tandis que l'autre bout est pourvu d'une tige de contact (9) en retrait d'une surface terminale (3) conçue pour contact avec la peau dans une zone d'acupuncture, ladite tige de contact étant reliée à une première électrode (19) d'un convertisseur piézoélectrique (18), dont la deuxième électrode est en connexion électrique, d'un côté, avec un contact à main (7) et est susceptible d'être commandée mécaniquement, de l'autre côté, au moyen d'un marteau de relieur à ressort (23) commandé par le bouton-poussoir d'actionnement (5) pour la génération d'une impulsion analgésique et électrique à haute tension avec un bas niveau de puissance, **caractérisé en ce que** le convertisseur piézoélectrique (18) est monté avec lesdites première et deuxième électrodes (19, 20) et ledit marteau de relieur (23) avec un système de ressort associé qui comprend un ressort de pression d'actionnement (25) et un ressort de retour (27) dans un fourreau intérieur électriquement isolant (8) conçu pour montage adapté à forme dans le fourreau extérieur essentiellement électriquement isolant (1) avec ladite tige de contact (9) faisant saillie à l'un bout du fourreau intérieur (8), à l'autre bout duquel est monté un activant à marteau de relieur (16) capable de déplacement longitudinal et étant relié mécaniquement au bouton-poussoir d'actionnement (5), la connexion électrique entre la deuxième électrode (20) du convertisseur piézoélectrique (18) et ledit contact à main (7) comprenant un contact de ressort à feuille (10) qui fait saillie à travers le fourreau intérieur (8) et s'étend entre ledit fourreau intérieur et le fourreau extérieur (1) pour établir un contact avec le contact à main (7) conçu comme une bague de contact.

2. Stimulateur destiné à l'acupuncture selon la revendication 1, **caractérisé en ce que** le contact de ressort à feuille (10) avec une portion de bout repliée (11) est fixé dans un évidement (12) au bout libre d'une extrémité (13) du fourreau extérieur (1), l'extrémité qui sert de support pour la bague de contact (7).

3. Stimulateur destiné à l'acupuncture selon la revendication 1 ou 2, **caractérisé en ce que** le bouton-poussoir d'actionnement (5) est monté dans une partie supérieure (4) placée en prolongation du fourreau extérieur (1) et de la bague de contact (7) et présentant un appui faisant saillie (6) comme support pour l'articulation de l'index de l'utilisateur lorsque le bouton-poussoir (5) est commandé par le pouce.
